(19)

**Europäisches Patentamt**
**European Patent Office**
**Office européen des brevets**

(11) **EP 4 259 827 B1**

(12) **EUROPEAN PATENT SPECIFICATION**

(45) Date of publication and mention
of the grant of the patent:
**01.01.2025 Bulletin 2025/01**

(51) International Patent Classification (IPC):
**C12Q 1/6876** (2018.01)  **A23L 33/00** (2016.01)

(21) Application number: **21824392.1**

(52) Cooperative Patent Classification (CPC):
**C12Q 1/6876**

(22) Date of filing: **10.12.2021**

(86) International application number:
**PCT/EP2021/085253**

(87) International publication number:
**WO 2022/123036 (16.06.2022 Gazette 2022/24)**

(54) **INCREASED 2'-FL PRODUCTION BY GOATS**

ERHÖHTE 2'-FL PRODUKTION DURCH ZIEGEN

PRODUCTION AUGMENTÉE DE 2'-FL PAR LES CHÈVRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.12.2020 EP 20386055
11.02.2021 EP 21156671**

(43) Date of publication of application:
**18.10.2023 Bulletin 2023/42**

(73) Proprietor: **AUSNUTRIA B.V.**
**8025 BM Zwolle (NL)**

(72) Inventors:
  • **HAANDRIKMAN, Alfred Jacques**
    **8025 BM Zwolle (NL)**
  • **HAPPE, Randolph Peter**
    **8025 BM Zwolle (NL)**
  • **PELLIS, Elisabeth Petronella Maria**
    **8025 BM Zwolle (NL)**
  • **BENJAMINS, Frédéric**
    **8025 BM Zwolle (NL)**
  • **DIJKHUIZEN, Lubbert**
    **9747 AG Groningen (NL)**
  • **CHATZIIOANNOU, Anastasia Chrysovalantou**
    **9747 AN Groningen (NL)**
  • **VAN LEEUWEN, Sander Sebastiaan**
    **9713 GZ Groningen (NL)**

(74) Representative: **V.O.**
**P.O. Box 87930**
**2508 DH Den Haag (NL)**

(56) References cited:
**WO-A1-99/11773**

• **MARTÍN-ORTIZ A ET AL: "Characterization of goat colostrum oligosaccharides by nano-liquid chromatography on chip quadrupole time-of-flight mass spectrometry and hydrophilic interaction liquid chromatography-quadrupole mass spectrometry", JOURNAL OF CHROMATOGRAPHY A, ELSEVIER, AMSTERDAM, NL, vol. 1428, 21 September 2015 (2015-09-21), pages 143 - 153, XP029378752, ISSN: 0021-9673, DOI: 10.1016/J.CHROMA.2015.09.060**
• **SOUSA YASMIM R F ET AL: "Goat milk oligosaccharides: Composition, analytical methods and bioactive and nutritional properties", TRENDS IN FOOD SCIENCE AND TECHNOLOGY, ELSEVIER SCIENCE PUBLISHERS, GB, vol. 92, 2 August 2019 (2019-08-02), pages 152 - 161, XP085817783, ISSN: 0924-2244, [retrieved on 20190802], DOI: 10.1016/J.TIFS.2019.07.052**
• **ANONYMOUS: "Transcript: FUT2-201 (ENSCHIT00000020504.1) - Variant table - Capra_hircus - Ensembl genome browser 104", 7 June 2021 (2021-06-07), XP055810777, Retrieved from the Internet <URL:http://www.ensembl.org/Capra_hircus/Transcript/Variation_Transcript/Table?db=core;g=ENSCHIG00000014375;r=18:56356093-56357127;t=ENSCHIT00000020504> [retrieved on 20210607]**

## Description

FIELD OF THE INVENTION

The invention disclosed herein relates to the field of production of dairy products; more specifically to a method for producing goat milk having naturally present 2'-fucosyllactose.

BACKGROUND

Human milk is considered to be the best food for new-borns and young infants and an important part of nutrition until at least the age of two (WHO and UNICEF recommend exclusive breastfeeding up to two years). Human milk comprises a complex mixture of nutrients and bioactive components thereby providing all nutrients the infant needs. Among the bioactive components, human milk oligosaccharides (hMOS) have been described to play an important role through their function as prebiotics and in inhibiting adhesion of pathogens and viruses to intestinal epithelial receptors. Over 160 hMOS structures have been described to be present in human milk in concentrations from around 20-25 g/L in colostrum and concentrations that decline to around 5-20 g/L in mature milk. Of the total amount of hMOS neutral fucosylated hMOS are predominant over acidic sialylated hMOS (50-70% over 10-25% respectively). In most people 2'fucosyllactose (2'-FL) is one of the most abundant hMOS in secretor mothers and in some cases constitutes nearly 30% of all hMOS. 2'-FL has been described to play an important role in immune function and protecting infants against gut infections by acting as a pathogen trap. Breast fed infants and infants fed 2'-FL supplemented formula show lower concentrations TNF-$\alpha$ and IFN-y when compared to non-2'-FL fed infants.

In human galactoside 2-alpha-L-fucosyltransferase 2 is an enzyme encoded by the dominant allele of the FUT2 gene, while the recessive allele does not produce a functional enzyme. Galactoside 2-alpha-L-fucosyltransferase 2 is necessary for the synthesis of 2'-FL and other fucosylated hMOS.

Intra individual variation in hMOS fucosylation is based on the maternal secretor and Lewis blood group status. The secretor (Se) gene encodes for the FUT2 enzyme which is necessary for the synthesis of 2'-FL and other fucosylated hMOS and is expressed in the lactating mammary gland. (Se+) secretor women produce milk with an abundance of $\alpha$1-2-fucosylated hMOS, especially 2'-FL. Non-secretor females, by contrast, lack the FUT2 enzyme. In the milk of those women only minimal amounts or no 2'-FL and other $\alpha$1-2-fucosylated hMOS are found. Approximately 80% of European and American women are found to be of the secretor type.

Although the importance of breastfeeding is stressed by the WHO, not all mothers are able or willing to breastfeed their baby. Infant formulae are the best choice when breastfeeding is no option. Infant formula manufacturers aim to produce infant formulae that are as close as possible to human breastmilk in composition and hence performance.

Most infant formulae are made from cows' or goats' milk, as they are easily available in large quantities. Interestingly, milk of these animals has been found to contain oligosaccharides like those found in human milk. However, the diversity of oligosaccharides present in cows' and goats' milk is limited. Over 160 structures of hMOS have been identified, compared to up to 40 for bovine milk and around 50 for goat milk.

Beneficial effects are attributed to hMOS in general, but also to individual hMOS structures. The most-reported individual hMOS studied are the 2'-FL, 3-FL, 3'-SL, 6'-SL, 3' GL, 6'-GL, LNFP II, LNT, LNnT and DFLNT. Interestingly, 2'-FL, which is one of the most abundant oligosaccharides found in secretors human milk, has been found in goats' milk but not in cows' milk.

The concentration of oligosaccharides in the milk of domestic mammals such as cows and goats are in general much lower than the concentration of oligosaccharides found in human milk, typically between 0.03 to 0.06 for cow and 0.25 to 0.5 g/L for goat. Likewise, the concentration of 2'-FL in goat milk is much lower than the concentration of 2'-FL in human milk. Thus, although goat milk is found to comprise 2'-FL, which makes it an interesting alternative to cow's milk when human milk is not available, the concentration of oligosaccharides in general and 2'-FL in particular is nowhere near the levels found in human milk.

No company is currently able to provide a regular infant formula with the diversity and concentration of oligosaccharides similar to those found in human milk. The use of synthetic oligosaccharides is limited due to the difficulty of synthesis and their high price. In addition, the use of synthetic oligosaccharides is strictly regulated in many jurisdictions, resulting in its limited use in regular formulae such as infant formula and the like. When used, synthetic oligosaccharides are often used in specialty formulae. EP2934190 for instance discloses a nutritional composition for use in modulating or reducing inflammation in an adult or elderly enriched with hMOS such as lacto-N-neotetraose and 2'-FL. WO2014100191 discloses nutritional compositions for use in providing a neuroprotective effect comprising additionally included oligosaccharides such as lacto-N-neotetraose and 2'-FL.

In the case of using natural oligosaccharide sources, up to 20% of total oligosaccharides may be lost during the isolation process.

The low levels of total oligosaccharides in milk of mammals other than human therefore remain a problem when seeking for a complete food for new-borns and young infants not able to feed on human milk.

The invention seeks to provide a goat milk that more closely approximates human breastmilk composition and performance. Document by SOUSA YASMIM R F ET AL: "Goat milk oligosaccharides: Composition, analytical methods and bioactive and nutritional properties", TRENDS IN FOOD SCIENCE AND TECHNOLOGY, ELSEVIER SCIENCE PUBLISHERS, GB, vol. 92, 2 August 2019 (2019-08-02), pages 152-161, discloses that goat milk is a source of 2'-fucosyllactose (2'-FL). Document by MARTÍN-ORTIZ A ET AL: "Characterization of goat colostrum oligosaccharides by nano-liquid chromatography on chip quadrupole time-of-flight mass spectrometry and hydrophilic interaction liquid chromatography-quadrupole mass spectrometry", JOURNAL OF CHROMATOGRAPHY A, ELSEVIER, AMSTERDAM, NL, vol. 1428, 21 September 2015 (2015-09-21), pages 143-153, discloses the results of determining the amounts of 2'-FL in milk of six different goats.

## SUMMARY OF THE INVENTION

In order to address one or more of the foregoing desires, the invention provides in one aspect a method of producing goat milk having naturally present 2'-Fucosyllactose (2'-FL) comprising

> a) determining whether a goat is producing 2'-FL;
> b) selecting a plurality of goats that produce 2'-FL; and
> c) milking the selected goats.

In a second aspect the disclosure invention provides a method which is not claimed, for determining whether a goat possesses at least one copy of a favourable allele at a SNP and/or has one copy with a deletion in the region of the FUT2 gene wherein the presence or absence of the polymorphism is determined by DNA sequencing using at least one of specific primers having SEQ ID No: 1 and SEQ ID No: 2 and/or SEQ ID No: 3 and SEQ ID No: 4 and/or SEQ ID No: 5 and SEQ ID No: 6.

In a third aspect the method of invention provides a goat milk obtainable by the method of producing the goat milk having naturally present 2'-fucosyllactose.

In a fourth aspect there is disclosed, but not claimed, a component obtained from the milk wherein the component is whey, whey protein isolate, whey protein concentrate, demineralised whey, casein, micellar casein concentrate, oligosaccharide and/or 2'-FL.

In a fifth aspect there is disclosed, but not claimed, a food product comprising the goat milk wherein the food product is a preterm infant formula, an infant formula, a follow up formula, an adult formula, a mama formula, a medical formula, a sports formula or a cream, yoghurt, butter, cheese or ice cream.

In a sixth aspect there is disclosed, but not claimed, a SNP marker associated with a 2'-FL production trait, characterised in that the SNP marker is located in the FUT2 gene and has a base mutation CC - CT at base pair 604 and/or CG - GT or TT at base pair 931.

## BRIEF DESCRIPTION OF THE DRAWING

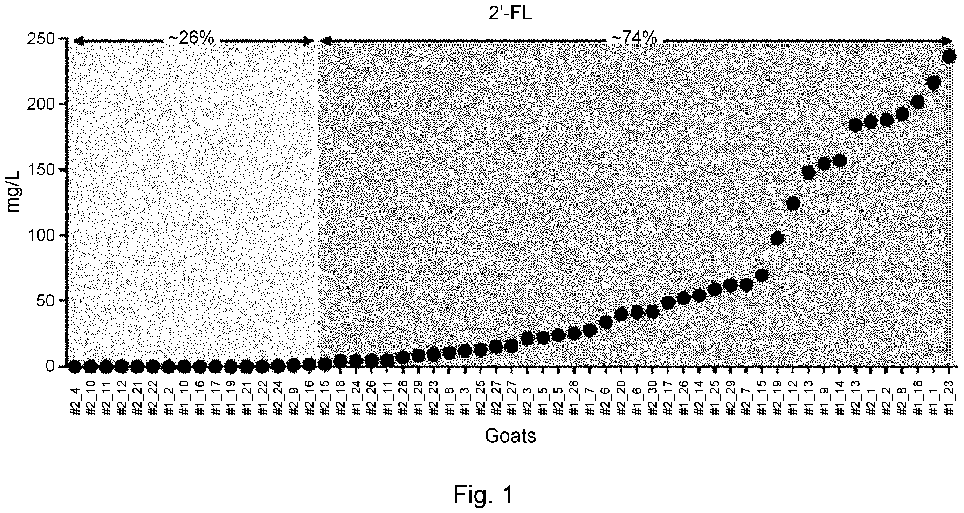

> fig. 1 Concentrations of 2'-FL in milk samples of a goat population.

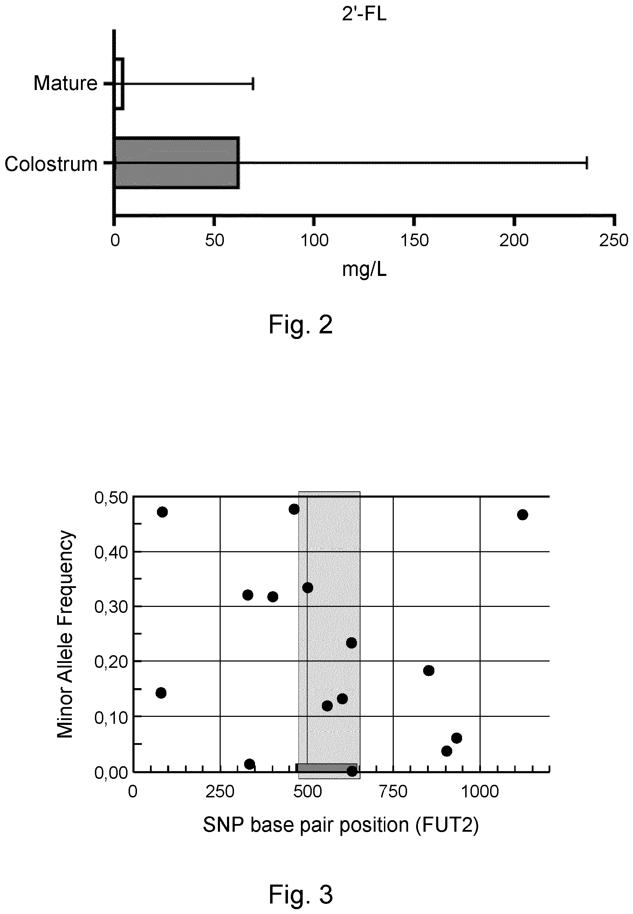

> fig. 2 Comparison of 2'-FL concentrations in goat milk.
> fig. 3 Localisation of SNPs and deletions in FUT2 gene.

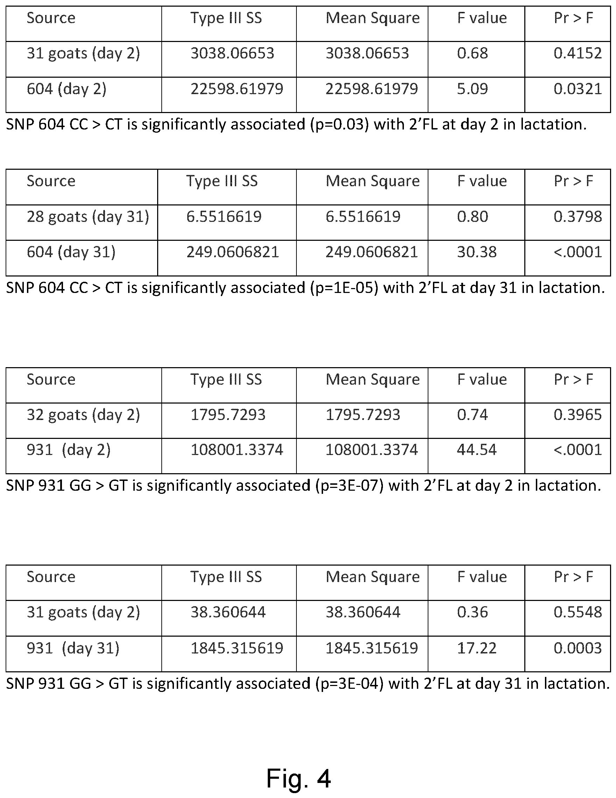

> fig. 4 SNP genotypes associated with 2'-FL

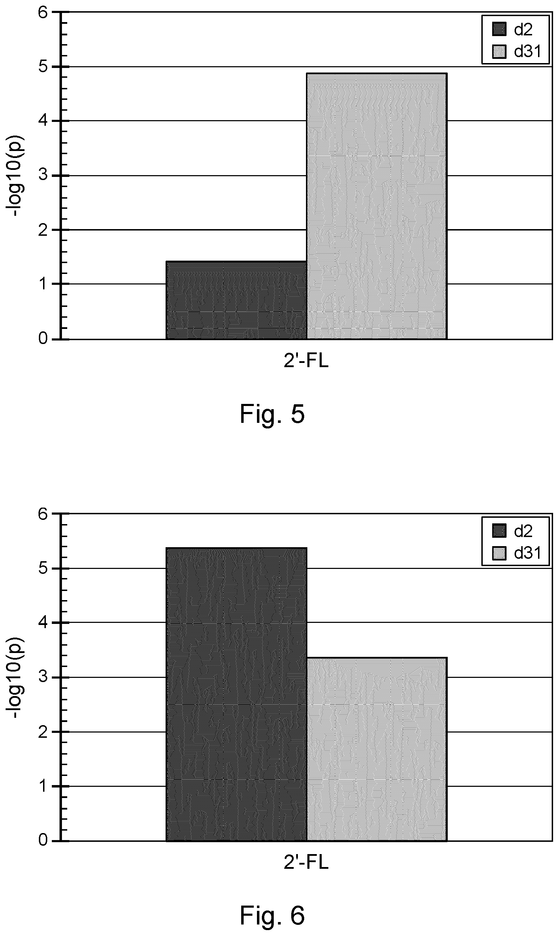

> fig. 5 Significant associations of SNP at bp 604 on 2'-FL concentration.
> fig. 6 Significant associations of SNP at bp 931 on 2'-FL concentration.

## DETAILED DESCRIPTION OF THE INVENTION

Definitions

Human milk oligosaccharides are one of the main components of human breast milk. Human breast milk contains about 5-25 g/L of human milk oligosaccharides which comprises on average, 10 g/L of neutral oligosaccharides and 1 g/L of acidic oligosaccharides. The composition of human milk oligosaccharides is very complex and currently over 160 different human oligosaccharide structures are known.

The term "human milk oligosaccharide" or "hMOS" as used herein, unless otherwise specified, refers generally to a number of complex carbohydrates found in human breast milk that can be in acidic or neutral form, and to precursors thereof. Exemplary non-limiting human milk oligosaccharides include 3'-sialyllactose (3'-SL), 6'-sialyllactose (6'-SL), lacto-N-neotetraose (LNnT), lacto-N-tetraose (LNT), disialylated lacto-N-tetraose (DSLNT), 3-fucosyllactose (3-FL), and 2'-fucosyllactose (2'-FL).

The term "goat milk oligosaccharide" or "gMOS" as used herein, unless otherwise specified, refers generally to a number of complex carbohydrates found in caprine or goat breast milk that can be in acidic or neutral form, and to precursors thereof. Exemplary non-limiting goat milk oligosaccharides include 3'-sialyllactose (3'-SL), 6'-sialyllactose (6'-SL), 3-fucosyllactose (3-FL), and 2'-fucosyllactose (2'-FL).

The term "colostrum" as used herein is the initial milk that is secreted by mammals which production starts before delivery and continues the first few days after giving birth, regularly until 5 days after delivery.

The term "mature milk" as used herein is the milk provided by the mammal from day 5 after parturition.

The terms "nutritional formulation" or "nutritional composition" as used herein, are used interchangeably and, unless otherwise specified, refer to formulae including nutritional liquids, nutritional powders, nutritional solids, nutritional semi-solids, nutritional semi-liquids, nutritional component, and any other nutritional food product as known in the art. The nutritional powders may be reconstituted to form a nutritional liquid, all of which are suitable for oral consumption by a human. The terms "nutritional formulation" or "nutritional composition" do not include human breast milk.

The term "nutritional powder" as used herein, unless otherwise specified, refers to nutritional products in scoop able form that can be reconstituted with water or another aqueous liquid prior to consumption and includes both spray dried and dry mixed/dry blended powders.

The term "nutritional liquid" as used herein, unless otherwise specified, refers to nutritional products in ready-to-drink liquid form, concentrated form, and nutritional liquids made by reconstituting the nutritional powders described herein prior to use.

The term "nutritional semi-solid" as used herein, unless otherwise specified, refers to nutritional products that are intermediate in properties, such as rigidity, between solids and liquids. Some semi-solids examples include, but are not limited to, puddings, gelatines, ice creams and doughs.

The term "nutritional semi-liquid," as used herein, unless otherwise specified, refers to nutritional products that are intermediate in properties, such as flow properties, between liquids and solids. Some semi-liquids examples include thick shakes and liquid gels.

The term "nutritional component" as used herein, unless otherwise specified, refers to components derived from further processing of the milk. Further processing is herein understood to comprise processes commonly used in the dairy processing industry such as, but not limited to, filtration steps such as nano-, ultra- or microfiltration, centrifugation, evaporation, crystallisation, coagulation and so forth. Components derived from these steps are known to comprise whey, whey protein isolate, whey protein concentrate, demineralised whey, casein, micellar casein concentrate, oligosaccharides such as, but not limited to, for instance 3'-sialyllactose (3'-SL), 6'-sialyllactose (6'-SL), 3-fucosyllactose (3-FL), and 2'-fucosyllactose (2'-FL).

The term "infant" or "term infant" as used herein, unless otherwise specified, refers to a person 12 months or younger.

The term "preterm infant" as used herein, refers to a person born prior to 36 weeks of gestation.

The term "newborn" as used herein, unless otherwise specified, refers to a person from birth up to four weeks of age.

The term "toddler" as used herein, unless otherwise specified, refers to a person greater than one year of age up to three years of age.

The term "child" as used herein, unless otherwise specified, refers to a person greater than three years of age up to twelve years of age.

The term "adult" as used herein, unless otherwise specified, refers to a person greater than 12 years of age.

The term "elderly" as used herein, refers to an individual of at least 45 years of age, including at least 50 years of age, at least 55 years of age, at least 60 years of age, at least 65 years of age, at least 70 years of age, at least 75 years of age, and including at least 80 years or age or greater.

The term "infant formula" as used herein, unless otherwise specified, is used interchangeably and refers to liquid, solid, semi-solid, and semi-liquid human milk replacements or substitutes that are suitable for consumption by an infant. The term "infant formula" does not include human breast milk.

The term "preterm infant formula" as used herein, unless otherwise specified, refers to liquid nutritional products suitable for consumption by a preterm infant.

The term "mama formula" as used herein, unless otherwise specified, refers to liquid, semi-liquid, semi-solid and solid nutritional products suitable for consumption by a female expecting or having given birth to a child.

The term "sports formula" as used herein, unless otherwise specified, refers to liquid, semi-liquid, semi-solid and solid nutritional products suitable for consumption by a person performing physical exercise.

The term "medical formula" as used herein, unless otherwise specified, refers to liquid, semi-liquid, semi-solid and solid nutritional products suitable for consumption by a person in medical need thereof.

Numerical ranges as used herein are intended to include every number and subset of numbers within that range, whether specifically disclosed or not. Further, these numerical ranges should be construed as providing support for a claim directed to any number or subset of numbers in that range. For example, a disclosure of from 1 to 10 should be construed as supporting a range of from 2 to 8, from 3 to 7, from 5 to 6, from 1 to 9, from 3.6 to 4.6, from 3.5 to 9.9, and so forth.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The invention involves producing goat milk comprising naturally present 2'-Fucosyllactose (2'-FL). Determining which goats produce 2'-FL is a first critical step in obtaining milk comprising naturally present 2'-FL. Determining what goats produce milk comprising naturally present 2'-FL can be done by techniques known by the person skilled in the art such as, but not limited to, measuring the amount of 2'-FL directly in the milk or via genetic means before milking the goats. The inventors believe it is the first time shown that 2'-FL is produced by only a selection of goats in a population. It was found that approximately 75% of the overall goat population tested produced 2'-FL while approximately 25% of the overall goat population tested did not produce 2'-FL. Additionally it was found that in goats producing 2'-FL the concentration of 2'-FL in the milk produced varies significantly between individual goats, making it possible to select goats producing higher concentrations of 2'-FL.

A second step is selecting a plurality of goats that produce the milk comprising naturally present 2'-FL. An advantage of selecting goats that produce milk comprising naturally present 2'-FL is that a pool of goats is created possessing a preferred trait such as milk comprising naturally present 2'-FL.

A third step is milking the selected goats that produce the milk comprising naturally present 2'-FL. An advantage of milking the selected goats that produce milk comprising naturally present 2'-FL separately from goats that do not have 2'-FL naturally present in the milk is that a pool of milk is formed with milk comprising 2'-FL, thus preventing dilution of 2'-FL over a larger amount of milk.

In a preferred embodiment the milk of the selected goats is collected for further processing. Collecting the milk is herein understood as milking the individual goats and adding the milk to a tank wherein the milk of a plurality of goats is gathered. The milk collected or gathered in a tank is also known as bulk milk. The bulk milk tank is used as a storage tank for cooling and holding the milk until it is used for further processing. Bulk milk tanks may hold an amount of bulk milk from about 150L to about 10000L such as 500L, 1000L, 2000L, 3000L, 4000L, 5000L, 600L, 7000L, 8000L or 9000L depending on the type of tank used. The person skilled in the art is acquainted with the different types of bulk milk tanks used for holding the milk for further processing. Depending on the type of further processing different bulk milk tanks may be used to collect and hold the milk. The person skilled in the art has knowledge on the amount of bulk milk needed to perform the further processing steps to achieve the aimed for end product. Depending on the end product different processing steps may be performed on the milk. Products made from goat milk are, but are not limited to, for instance pasteurised goat milk, goat yoghurt, goat cheese, goat milk based nutritional formulae such as infant formula, pre-term infant formula, toddler formula, mama formula, elderly formula, sports formula and medical formula. To produce these products the goat milk for instance has to be pasteurised, sterilised, micro-filtrated, ultra-filtrated, nano-filtrated, coagulated, spray dried. The person skilled in the art is capable of calculating the amount of bulk milk needed for performing the necessary steps to further process the milk to achieve a product made from the goat milk comprising naturally present 2'-FL.

When producing products from goat milk such as (pasteurised) goat milk, goat yoghurt, goat cheese, ice cream and the like the person skilled in the art will use a minimum of about 50, 60, 60, 80, 90 100, 120, 140 or 150L of goat milk to produce at a commercial scale. When producing for larger commercial scale the volume used may be from 5000L up to 10 000L or more. When producing nutritional formula such as, but not limited to, infant-, follow-up-, mama-, adult-, elderly-, sports- or medical formula at commercial scale the minimal volume necessary, to produce, is at least 4000 L. Preferably the volume used is at least 5000L, 6000L, 7000L, 8000L, 9000L or 10 000L or more. The person skilled in the art reckons it is possible to produce these formulae at smaller scale such as around 1000L, 2000L or 3000L to produce milk powder when using spray drying equipment such as the Filtermat spray drying tower from Amtech or similar equipment.

In a preferred embodiment the goat milk is colostrum, mature milk or a combination thereof. Different types of goat milk such as colostrum, mature milk or a combination thereof may be collected and used for further processing. Colostrum is the initial milk that is secreted by mammals which production starts just before delivery and continues the first few days after giving birth, regularly until 5 days after delivery. The composition of colostrum is known to differ from mature milk in for instance composition, nutritional value and ability to boost the immune system of the neonate. In general, colostrum is known to have a mild laxative effect. Colostrum comprises antibodies such as IgA, IgG, and IgM and immune cells such as lymphocytes that protect the new born against diseases. The transition of colostrum to mature milk happens around day five after parturition. In general, protein concentration in colostrum is high but lowers when transitioning to mature milk. Other components such as specific antibodies including IgA, IgG, and IgM, cytokines, growth factors including insulin-like growth factors also decrease during these first five days after parturition. Use of colostrum or addition of colostrum to goat milk products may have health benefits for the consumer. As the timing of changing from colostrum to mature milk is well known in the art it is possible to selectively milk goats that are producing colostrum or goats that produce mature milk. As colostrum is known to comprise higher concentrations of oligosaccharides and higher concentration of specific oligosaccharides such as 2'-FL this knowledge provides the opportunity to selectively identify goats that produce colostrum. Of these colostrum producing goats, it is determined which goats produce 2'-FL. These 2'-FL producing goats are selected and milked. The milk of these goats is then collected, preferably in bulk, to be further processed. Not all farmers however milk goats that produce colostrum. At these farms goats producing mature milk will be identified and selected. Of these mature milk producing goats, it is determined which goats produce 2'-FL. These 2'-FL producing goats are selected and milked. The milk of these goats is then collected, preferably in bulk, to be further processed. Other farms will milk and collect the milk of both the colostrum producing goats and the mature milk producing goats.

The use of colostrum into products such as pre-term infant formula and infant formula is regulated in many jurisdictions. For many other products such as sports formula and adult formula however, regulations are less strict or even absent, creating possibilities to make use of the beneficial properties of colostrum such as improving athlete performance and decreasing recovery time after work out.

Due to the different aspects regarding intention and the allowed use as described here above it is interesting to collect the colostrum separately from the mature milk. Based on the type of product preferred the person skilled in the art may combine colostrum and mature milk to obtain a product such as for instance described here above.

In a preferred embodiment the concentration of naturally present 2'-FL in the collected mature milk is least 13 mg/L. Milk is considered mature milk from five days after parturition. Although the composition of mature milk changes over time to the needs of the child, the mature milk remains relatively stable over a longer period of time when compared to the rapid changes in composition of the colostrum . Mature milk is the main milk used for consumption and further processing as it can be milked from the goat for a longer period of time.

In a preferred embodiment the concentration of naturally present 2'-FL in the collected mature milk is at least 15 mg/L, such as at least 30 mg/L, such as at least 60 mg/L, such as at least 70 mg/L, such as at least 80 mg/L. Preferably the collected mature milk comprises a naturally present 2'-FL concentration as high as possible such as at least 60 mg/L, at least 70 mg/L, or at least 80 mg/L.

In a preferred embodiment the concentration of naturally present 2'-FL in the collected colostrum is at least 100 mg/L. The colostrum can be collected from just before giving birth until 5 days after giving birth. During these first days after delivery the concentration of 2'-FL steadily decreases until it reaches the concentrations found in mature milk.

In a preferred embodiment the concentration of naturally present 2'-FL in the collected colostrum is at least 125 mg/L, such as at least 150 mg/L, such as at least 175 mg/L, such as at least 200 mg/L, such as at least 220 mg/L, such as at least 240 mg/L, such as at least 260 mg/L, such as at least 280 mg/L or at least 300 mg/L.

In a preferred embodiment the collected colostrum and collected mature milk are mixed in a ratio of 99:1 to 1:99, such as 90:10 to 10:90, such as 80:20 to 20:80, such as 70:30 to 30:70, such as 60:40 to 40:60 or 50:50. Colostrum and mature milk may be mixed directly when collecting the milk from a population of goats that are at different stages after parity and thus the population holds goats that produce both colostrum and mature milk. Another way is to first select either colostrum producing goats and mature milk producing goats, milk these separately and at a later timepoint mix the collected milk in a specific ratio from 1:99 to 99:1 to obtain a preferred mixture. Depending on the type of product to obtain, the person skilled in the art will be able to mix the collected colostrum with the collected mature milk to obtain a colostrum/ mature milk mixture comprising the specifically aimed for 2'-FL concentration.

In an aspect of the disclosure it is determined whether the goat producing 2'-FL possesses at least one favourable allele at a SNP and/or has one copy with a deletion in the region of the FUT2 gene. A favourable allele herein means the presence of an allele that results in the production of higher 2'-FL concentration naturally present in the milk when compared to milk from goats that do not have this allele. The goat FUT2 gene is located on chromosome 18 (ENSCHIG00000014375). Surprisingly it has been found that specific Single Nucleotide Polymorphisms (SNP) and or deletions in the region of the FUT2 gene are correlated to the concentration of naturally present 2'-FL found in goats milk. A large variation of 2'-FL concentration was found in different goats producing 2'-FL which were related to different SNPs and/or a deletion within the FUT2 gene.

A first measure is to determine whether the goat producing 2'-FL possesses at least one copy of a favourable allele at a SNP and/or has one copy with a deletion in the region of the FUT2 gene that results in a higher concentration of 2'-FL naturally present in the milk compared to milk from goats that do not have at least one favourable allele at the SNP and/or one copy with a deletion in the region of the FUT2 gene. Milk of goats that do not have the at least one favourable allele at the SNP and/or one copy with a deletion in the region of the FUT2 gene contains typically between 1 to 5 mg/L of 2'-FL.

A second measure is to select a plurality of goats that have the at least one favourable allele at the SNP and/or one copy with a deletion in the FUT2 gene.

A third measure is to milk the selected plurality of goats that have the at least one favourable allele at the SNP and/or one copy with a deletion in the FUT2 gene. An advantage of selecting and milking the goats that have a higher concentration of 2'-FL naturally present in the milk, separately from goats that do not have 2'-FL naturally present in the milk or have lower concentrations of 2'-FL naturally present in the milk, is that a pool of milk is formed only with milk comprising 2'-FL, preferably with higher concentrations of 2'-FL, thus preventing dilution of 2'-FL over a larger amount of milk.

The inventors therefore believe that selecting goats that produce 2'-FL and preferably selecting goats that produce higher concentrations of 2-'FL will result in an increased concentration of 2'-FL in the total bulk milk. Bulk milk herein is the milk collected from a plurality of goats and pooled and stored in a bulk milk tank.

In a preferred embodiment the goat milk is from a goat having at least one favourable allele at the SNP that is located at any of or a combination of base pair 604 and 931. The favourable allele at the SNPs found showed to have an effect on the concentration of 2'-FL in the goat milk. Other variant copies of alleles were found at base pair 77, 84, 330, 335, 464, 504, 558, 629, 853, 904, 1123. These can be combined with any of the at least one favourable allele at the SNP 604 and/or 931.

In a preferred embodiment the goat milk is from a goat having the at least one favourable allele at the SNP that is located at any of or a combination of base pair 604 and/or 931 and wherein the base pair variants of the polymorphism at base pair 604 is CC to CT and at base pair 931 is GG to GT or TT.

Other variant copies of alleles were found at base pair 77, 84, 330, 335, 464, 504, 558, 604, 629, 853, 904, 931, 1123. The base pair variants of these polymorphisms are:

    a. 77 CC - CT
    b. 84 CC - CT or TT
    c. 330 GG to AG or AA
    d. 335 CC - CT
    e. 463 GG - AG or AA
    f. 504 AA - AC or CC
    g. 558 GG
    h. 629 CG - CC or GG
    i. 853 TT - CT
    j. 904 CC - CG
    k. 1123 CT - TT or CC

In a preferred embodiment the deletion is in the region of base pair position of around 375 to around 750, preferably in the region of base pair position 401 to 631. The deletion may lead to loss the base pairs 463, 504, 558, 604, 629.

Goats with a deletion in the FUT2 gene can have high 2'-FL levels when the deletion is combined with a base pair variant of any or more of the base pairs 604 and/or 931 at the other allele.

In a preferred embodiment the favourable allele at the SNP is located at base pair 931 but not at base pair 604. It was found that goats having a favourable allele at the SNP at only base pair 931 were having the highest amount of naturally present 2'-FL in their milk. Selecting goats with only this favourable allele at the SNP and not the deletion or the favourable allele SNP at base pair 604 may therefore lead to highest concentration of 2'-FL in the collected milk.

In a preferred embodiment the favourable allele at the SNP at base pair 931 is GG - GT or TT.

In another aspect the disclosure involves a method for determining whether a goat possesses the at least one favourable allele at the SNP in the and/or has a deletion in the region of the FUT2 gene wherein the presence or absence of the polymorphism is determined by DNA sequencing using at least one of specific primers having SEQ ID No: 1 and SEQ ID No: 2 and/or SEQ ID No: 3 and SEQ ID No: 4 and/or SEQ ID No: 5 and SEQ ID No: 6.

In a preferred embodiment the method which is not claimed, for determining whether a goat possesses the at least one favourable allele at the SNP in the region of the FUT2 gene comprises the following steps:

    a) taking hairs of the goats to be tested and extracting its genomic DNA;
    b) performing a PCR amplification using at least one of the specific primers to obtain an amplified product fragment;
    c) detecting the base species at the 604 and/or 931 base pair product fragments.

Wherein the hairs of the goats to be tested must contain the hair follicle. Preferably at least 1 hair, 2 hairs, 3 hairs, 4 hairs or 5 hairs or more are taken from the goat for DNA extraction. The person skilled in the art is acquainted with techniques available to extract genomic DNA and perform PCR amplification techniques to obtain the amplified product fragment as well as with techniques to detect the base species at the specific base pair product fragments.

In another aspect the disclosure involves a goat milk obtainable by the method of producing goat milk having naturally present 2'-FL. The goat milk having naturally present 2'-FL may be consumed directly or be used to make further products wherein the naturally present 2'-FL provides nutritional advantages to the consumer such as improved immune function and protection against gut infections.

In a preferred embodiment the goat milk obtained by the method of producing goat milk having naturally present 2'-FL is whole milk, semi-skimmed milk or skimmed milk. Whole goat's milk contains approximately 3.5% fat (3.5g in 100mL of milk), semi skimmed goat's milk contains approximately 1.5% fat (1.5g in 100mL of milk) while skimmed goat's milk contains approximately 0.1% fat (0.1g in 100mL of milk). The whole milk, semi-skimmed milk or skimmed milk having naturally present 2'-FL may either be consumed directly or be further processed. Goat milk formulae such as preterm infant formula, infant formula, follow up formula, adult formula, elderly formula, mama formula, medical formula, sports formula may be produced from either the whole milk, semi-skimmed milk, skimmed milk having naturally present 2'-FL or any combination of these.

In a preferred embodiment the goat milk obtained by the method of producing goat milk having naturally present 2'-FL is having at least 13, 30, 60, 70 or 80 mg/L naturally present 2'-FL. The goat milk having naturally present 2'-FL having at least 13, 30, 60, 70 or 80 mg/L naturally present 2'-FL may be whole milk, semi-skimmed milk or skimmed milk. The whole milk, semi-skimmed milk or skimmed milk having at least 13, 30, 60, 70 or 80 mg/L naturally present 2'-FL may either be consumed directly or be further processed. Goat milk formulae such as preterm infant formula, infant formula, follow up formula, adult formula, elderly formula, mama formula, medical formula, sports formula may be produced from either the whole milk, semi-skimmed milk, skimmed milk having at least 13, 30, 60, 70 or 80 mg/L naturally present 2'-FL or any combination of these.

In another aspect there is disclosed, but not claimed, a component obtained from goat milk having naturally present 2'-FL wherein the component is whey, whey protein isolate, whey protein concentrate, demineralised whey, casein, micellar casein concentrate, oligosaccharide and/or 2'-FL. Goat milk is often further processed into components that may be added to or combined with other components. Whey and casein for instance are very important components in infant formula. In human breastmilk the whey:casein ratio is 60:40 and therefore considered a gold standard. In goat milk the natural whey:casein ratio is 20:80. When producing nutritional formulations such as for instance, but not limited to, infant formula the whey:casein ratio needs to be adjusted and therefore components may be separately obtained from goat milk. Casein and whey thus both need to be isolated to allow adjustment of the whey:casein ratio to 60:40. Other nutritional formulations such as for instance, but not limited to, mama formula, formula for elderly, medical formula or sports formula may require different whey:casein ratios. During processing the physicochemical properties of the components may change as they are subject to filtration steps such as microfiltration, ultrafiltration, nanofiltration. When using goat milk having naturally present 2'-FL in these steps, and preferably goat milk having high concentrations of naturally present 2'-FL the concentration of naturally present 2'-FL in the end product can be maintained. Further, components obtained from goat milk having naturally present 2'-FL are very important when legislation prohibits the use of synthetic or microbial oligosaccharides. Extracting the oligosaccharides such as 2'-FL from the goat milk and then using them on their own or as a component in for instance, but not limited to, nutritional formula such as a preterm infant formula, an infant formula, a follow up formula, an adult formula, an elderly formula, a mama formula, a medical formula, a sports formula is less restricted under existing legislation.

In another aspect there is disclosed, but not claimed, a food product obtained from goat milk having naturally present 2'-FL wherein the food product is a preterm infant formula, an infant formula, a follow up formula, an adult formula, a mama formula, an adult formula, a medical formula, a sports formula, cream, yoghurt, butter, cheese, ice cream or any other product made from milk known by the person skilled in the art. Infants fed with 2'-FL show higher amounts of Bifidobacterium and Bacteroides (which plays a role in processing complex molecules) compared to non-2'-FL fed infants. Further, 2'-FL is responsible for a broader range of bifidobacterial species as *Bifidobacterium longum subsp. infantis* dominated in 2'-FL-fed infants, but not in non-2'-FL fed infants. Providing an infant formula with an as high as possible naturally present 2'-FL is beneficial for a healthy gut microbiome development.

In a further aspect there is disclosed, but not claimed, a SNP marker associated with a 2'-FL production trait, characterised in that the SNP marker is located in the FUT2 gene and has a base mutation CC - CT at base pair 604 and/or CG - GT or TT at base pair 931.

In a preferred embodiment the SNP marker associated with a 2'-FL production trait is characterised in that it is significantly correlated with the 2'-FL concentration in goat milk. If the base of the SNP at base pair 604 is CT and/or base of the SNP at base pair 931 is GT or TT the production of 2'-FL by the animal is increased and therefore the concentration of 2'-FL in the milk is considerably higher than in animals lacking the base pair variant.

The aforementioned primers have the following nucleotide sequences (Forward (F) and reverse (R) primers indicated).

| SEQ ID No: 1 | CCCATCTTCCGAATCACACT | FUT2_SNP8_F |
|---|---|---|
| SEQ ID No: 2 | GTTGGGCATAACGTGGACTT | FUT2_SNP8_R |
| SEQ ID No: 3 | GGTGTCGGGAAAACATCAAC | FUT2_SNP12_F |
| SEQ ID No: 4 | TTGAGGAAGGGAGAGTCTGG | FUT2_SNP12_R |
| SEQ ID No: 5 | ACAAGCACATATTGCCCACA | FUT2_over_EXON_F |
| SEQ ID No: 1 | CCCATCTTCCGAATCACACT | FUT2_SNP8_F |

The invention will be further illustrated with reference to the following non-limiting examples.

EXAMPLES

Goat milk was obtained from Dutch goat farms. Milk samples of ~10 mL were collected from the goats during a full milking cycle starting from the day of birth until 10 months. A series of experiments was performed to achieve efficient milk oligosaccharide (MOS) extraction and labelling. These experiments were performed on pooled mature milk and on pooled colostrum. Direct labelling to 20 $\mu$L of reference milk was performed, following the Austin et al. [20] approach for human milk. Further tests were performed directly by centrifugation (21130 g for 30 min) of pooled mature milk and colostrum. After removal of excess lactose, extracted and freeze-dried oligosaccharides were labelled with the fluorophore 2-aminobenzamide (0.7 M, 2-AB) catalysed with 1 M 2-picoline-borane according to [22]. This labelling mix was prepared freshly in 7:3 Dimethyl sulfoxide (DMSO)/ glacial acetic acid solution; 50 $\mu$L labelling mix was added per sample, vortexed, centrifuged and incubated for 2 h at 65 °C.

The labelled and purified oligosaccharides were analysed using an Ultimate 3000 UHPLC system (Thermo Fischer Scientific) coupled to a FP-920 Fluorescence detector (Jasco Inc). The detector was set for excitation and emission at 330 nm and 420 nm, respectively.

The chromatographic separation was conducted on an Acquity UPLC Glycan BEH Amide column (2.1 mm x 100 mm, 130Å, 1.7 $\mu$m) and an Acquity UPLC Glycan BEH Amide VanGuard pre-column (2.1 mm x 5 mm, 130Å, 1.7 $\mu$m) both from Waters. The column was maintained at 40 °C. The 10 times diluted sample (2 $\mu$L) was injected in the system, under gradient elution with a quaternary solvent system. The solvent system used consisted of Acetonitrile (solvent A), 250 mM formic acid in 10% Acetonitrile in Milli-Q (pH 3.0) adjusted with ammonia (solvent B) and 10% Acetonitrile in Milli-Q (solvent C). The method used for the analysis was based on a gradient where solvent B was constant at 5% throughout and for 40 min the gradient was moving from 5% to 27% solvent C. The gradient was followed by a cleaning step with 20% solvent B and 20% solvent C for 5 min and a subsequent equilibration step to the initial conditions of the analysis for 12 min, resulting in a total analysis time of 57 min. The flow applied for optimal chromatographic separation was 0.5 mL/min.

Quantification was performed using maltopentaose as an internal standard and general calibrant, based on the study of Austin et al. [20]. This compound was selected, instead of the maltotriose in Austin's study, as it was eluting in an area of the chromatogram free of peaks for both mature and colostrum milk samples.

A commercial standard for 2'-FL was used for identification of MOS. The same chromatographic system and fluorescence detector were coupled in-line with a Time-of-Flight mass spectrometer (MaXis Plus, Bruker). The MS was operated in positive ion mode, scanning between m/z 300-2000, with collision energy 7 eV, collision RF 80 Vpp and transfer time 100 $\mu$s.

In order to monitor the quality of both the analysis and the sample preparation, reference goat milk samples were treated in the same way as the real milk samples, and were analysed in batches of 10-15 samples each [24,25].

By using the above described techniques, the 2'-FL structure was detected in the milk of 73.7% of the overall goat population. Among the goats producing milk having naturally present 2'-FL, there is a distribution of 2'-FL concentrations between 0.6 - 236.3 mg/L in the colostrum samples collected at Day 2. At the Day 31 time point, the range of 2'-FL concentrations was less diverse, ranging from 0.4 - 69.5 mg/L.

To determine whether the genotype of goats has an effect on the presence or absence of 2'FL production the DNA of goats was examined. Thereto DNA was extracted from the hair samples of both male and female goats and analysed for polymorphisms. Although milk is only produced by female goat both males and females could be carrying the genetic information for this trait. By determining whether there is a genetic trait involved in the production of 2'-FL selective breeding could be used to obtain a population of goat producing naturally present 2'-FL in their milk.

The DNA collected from the goats hair was sequenced and successfully genotyped showing various polymorphisms.

Several copies of different alleles were detected in the FUT2 gene. Further, goats were detected having one copy having a deletion in the FUT2 gene region. Variations of the SNPs were found at base pairs 77, 84, 330, 335, 464, 504, 558, 604, 629, 853, 904, 931, 1123. The base pair variants that were detected are:

a. 77 CC - CT
b. 84 CC - CT or TT
c. 330 GG - AG or AA
d. 335 CC - CT
e. 463 GG - AG or AA
f. 504 AA - AC or CC
g. 604 CC - CT
h. 629 CG - CC or GG
i. 853 TT - CT
j. 904 CC - CG
k. 931 GG - GT or TT
l. 1123 CT - TT or CC

The polymorphisms were then compared to the amount of 2'-FL present in both the colostrum (day 2) and the mature milk (day 31) of the goat. The data was analysed using the following model:

$$Y_{ij} = \mu + Farm_i + SNP_j + e_{ij}$$

Where

$Y_{ij}$ = the content 2'-FL content at day 2 or day 31 in milk
$\mu$ = overall mean
$Farm_i$ = the effect of Farm (i=1)
$SNP_j$ = the effect of SNP genotype (or deletion)
$e_{ij}$ = residual

Analyses were performed in SAS 9.4.
The analysis showed that variants of SNP 604 and 931 were significantly associated with 2'-FL content in milk, both in the colostrum of day 2 and in the mature milk of day 31.

**Claims**

1. A method of producing goat milk having naturally present 2'-fucosyllactose (2'-FL) comprising

a) determining whether a goat is producing 2'-FL;
b) selecting a plurality of goats that produce 2'-FL; and
c) milking the selected goats separately from goats that do not have 2'-FL naturally present in the milk.

2. The method of claim 1 further comprising collecting the milk of the selected goats as bulk milk for further processing, the bulk milk preferably being at least 150L.

3. The method of claim 2 wherein the bulk milk is at least 1000L, preferably at least 5000L most preferably at least 10,000L.

4. The method of claim 2 or 3 wherein the goat milk is mature milk.

5. The method of claim 2 or 3 wherein the goat milk is colostrum.

6. The method of claim 2 or 3 wherein the goat milk is a combination of mature milk and colostrum

**Patentansprüche**

1.  Verfahren zum Herstellen von Ziegenmilch mit natürlich vorhandener 2'-Fucosyllactose (2'-FL), umfassend:

    a) Bestimmen, ob eine Ziege 2'-FL produziert;
    b) Auswählen einer Vielzahl von Ziegen, die 2'-FL produzieren; und
    c) Melken der ausgewählten Ziegen, getrennt von Ziegen, die2'-FL nicht auf natürliche Weise in der Milch haben.

2.  Verfahren nach Anspruch 1, ferner umfassend das Auffangen der Milch der ausgewählten Ziegen als Sammelmilch zur weiteren Verarbeitung, wobei die Sammelmilch vorzugsweise mindestens 150 l beträgt.

3.  Verfahren nach Anspruch 2, wobei die Sammelmilch mindestens 1000 l, vorzugsweise mindestens 5000 l und am stärksten bevorzugt mindestens 10.000 l beträgt.

4.  Verfahren nach Anspruch 2 oder 3, wobei die Ziegenmilch reife Milch ist.

5.  Verfahren nach Anspruch 2 oder 3, wobei die Ziegenmilch Kolostrum ist.

6.  Verfahren nach Anspruch 2 oder 3, wobei die Ziegenmilch eine Kombination aus reifer Milch und Kolostrum ist.

**Revendications**

1.  Méthode de production de lait de chèvre présentant du 2'-fucosyllactose (2'-FL) naturellement présent comprenant

    a) la détermination du fait qu'une chèvre produit ou non du 2'-FL ;
    b) la sélection d'une pluralité de chèvres qui produisent du 2'-FL ; et
    c) la traite des chèvres sélectionnées séparément des chèvres qui ne présentent pas de 2'-FL naturellement présent dans le lait.

2.  Méthode selon la revendication 1 comprenant en outre la collecte du lait des chèvres sélectionnées sous forme de lait en vrac en vue d'un traitement ultérieur, le lait en vrac étant de préférence d'au moins 150 l.

3.  Méthode selon la revendication 2 dans laquelle le lait en vrac est d'au moins 1 000 l, de préférence d'au moins 5 000 l, de manière préférée entre toutes d'au moins 10 000 l.

4.  Méthode selon la revendication 2 ou 3 dans laquelle le lait de chèvre est du lait parfait.

5.  Méthode selon la revendication 2 ou 3 dans laquelle le lait de chèvre est du colostrum.

6.  Méthode selon la revendication 2 ou 3 dans laquelle le lait de chèvre est une combinaison de lait parfait et de colostrum.

Fig. 1

2'-FL

Fig. 2

SNP base pair position (FUT2)

Fig. 3

| Source | Type III SS | Mean Square | F value | Pr > F |
|---|---|---|---|---|
| 31 goats (day 2) | 3038.06653 | 3038.06653 | 0.68 | 0.4152 |
| 604 (day 2) | 22598.61979 | 22598.61979 | 5.09 | 0.0321 |

SNP 604 CC > CT is significantly associated (p=0.03) with 2'FL at day 2 in lactation.

| Source | Type III SS | Mean Square | F value | Pr > F |
|---|---|---|---|---|
| 28 goats (day 31) | 6.5516619 | 6.5516619 | 0.80 | 0.3798 |
| 604 (day 31) | 249.0606821 | 249.0606821 | 30.38 | <.0001 |

SNP 604 CC > CT is significantly associated (p=1E-05) with 2'FL at day 31 in lactation.

| Source | Type III SS | Mean Square | F value | Pr > F |
|---|---|---|---|---|
| 32 goats (day 2) | 1795.7293 | 1795.7293 | 0.74 | 0.3965 |
| 931 (day 2) | 108001.3374 | 108001.3374 | 44.54 | <.0001 |

SNP 931 GG > GT is significantly associated (p=3E-07) with 2'FL at day 2 in lactation.

| Source | Type III SS | Mean Square | F value | Pr > F |
|---|---|---|---|---|
| 31 goats (day 2) | 38.360644 | 38.360644 | 0.36 | 0.5548 |
| 931 (day 31) | 1845.315619 | 1845.315619 | 17.22 | 0.0003 |

SNP 931 GG > GT is significantly associated (p=3E-04) with 2'FL at day 31 in lactation.

# Fig. 4

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2934190 A

- WO 2014100191 A

**Non-patent literature cited in the description**

- Goat milk oligosaccharides: Composition, analytical methods and bioactive and nutritional properties. **SOUSA YASMIM R F et al.** TRENDS IN FOOD SCIENCE AND TECHNOLOGY. ELSEVIER SCIENCE PUBLISHERS, 02 August 2019, vol. 92, 152-161

- Characterization of goat colostrum oligosaccharides by nano-liquid chromatography on chip quadrupole time-of-flight mass spectrometry and hydrophilic interaction liquid chromatography-quadrupole mass spectrometry. **MARTÍN-ORTIZ et al.** JOURNAL OF CHROMATOGRAPHY A. ELSEVIER, 21 September 2015, vol. 1428, 143-153